# EUROPEAN PATENT SPECIFICATION

(11) **EP 2 142 646 B1**
(45) Date of publication and mention of the grant of the patent: **19.11.2014**
(21) Application number: 08734303.4
(22) Date of filing: 14.04.2008
(51) Int. Cl.: C12N 9/88, C07J 73/00

(54) **METHOD OF INCREASING OF THE PHOTOSYNTHETIC CARBON DIOXIDE ASSIMILATION YIELD**
VERFAHREN ZUR ERHÖHUNG DER PHOTOSYNTHETISCHEN KOHLENDIOXID-ASSIMILIERUNGSAUSBEUTE
PROCÉDÉ D'AUGMENTATION DU RENDEMENT D'ASSIMILATION DU DIOXYDE DE CARBONE PHOTOSYNTHÉTIQUE

(30) Priority: 17.04.2007 CZ 20070275
(43) Date of publication of application: 13.01.2010
(73) Proprietor: Ustav Organicke Chemie A Biochemie Akademie Ved Ceske Republiky, 166 10 Praha 6 (CZ)
(72) Inventor: MACEK, Tomás, 186 00 Praha 8 (CZ); UHLÍK, Ondrej, 251 66 Mirosovice (CZ); KAMLAR, Marek, 789 01 Zábreh (CZ); HARMATHA, Juraj, 160 00 Praha 6 (CZ); KOHOUT, Ladislav, 163 00 Praha 6 (CZ)
(74) Representative: Herman, Vaclav
(86) International application number: PCT/CZ2008/000044
(87) International publication number: WO 2008/125069

(56) References cited:
- YU JING QUAN ET AL: "A role for brassinosteroids in the regulation of photosynthesis in Cucumis sativus" JOURNAL OF EXPERIMENTAL BOTANY, vol. 55, no. 399, May 2004 (2004-05), pages 1135-1143, XP002505991 ISSN: 0022-0957
- MACHACKOVA VVANA MARTIN VAGNER ET AL: "Comparison between the effects of 20-hydroxyecdysone and phytohormones on growth and development in plants" EUROPEAN JOURNAL OF ENTOMOLOGY, vol. 92, no. 1, 1995, pages 309-316, XP002506032 ISSN: 1210-5759
- S. ÇA?, N. GÖREN-SA?LAM, Ç. ÇINGIL-BARI? AND E. KAPLAN: "THE EFFECT OF DIFFERENT CONCENTRATION OF EPIBRASSINOLIDE ON CHLOROPHYLL, PROTEIN AND ANTHOCYANIN CONTENT AND PEROXIDASE ACTIVITY IN EXCISED RED CABBAGE (BRASSICA OLERACEAE L.) COTYLEDONS" BIOTECHNOLOGY & BIOTECHNOLOGICAL EQUIPMENT, vol. 24, no. 4, 2007, XP002506033 [retrieved on 2008-11-28]
- O.UHLIK ET AL.: "Affinity chromatography reveals RuBisCo as an ecdysteroid-binding protein" STEROIDS, vol. 73, 2008, pages 1433-1440, XP002505992

## Description

### Technical field of invention

This invention deals with a method of increasing the yield of photosynthetic assimilation of carbon dioxide (CO₂) in plants by means of application of agents that increase the carboxylase activity of the enzyme ribulose 1,5-bisphosphate carboxylase/oxygenase (RuBisCO, EC 4.1.1.39).

### Background of the invention

Probably the most abundant protein of the biosphere on Earth, the enzyme ribulose 1,5-bisphosphate carboxylase/oxygenase (RuBisCO, EC 4.1.1.39) is the only enzyme able to prepare biomass in plants from inorganic compounds. It is present in high concentration in chloroplasts and represents up to 50 % of the soluble leaf proteins. During photosynthesis the energy of light is transformed to chemical energy, oxygen is released from water and the gaseous carbon dioxide from the air is fixed into organic compounds. In consequent steps are formed sugars, amino acids, fatty acids and other parts of living mass. RuBisCO is a unique and very interesting enzyme. It can be distinguished as the most important enzyme within the photosynthesis, catalyzing the key reaction of the Calvin cycle, so called carboxylation, during which the CO₂ is built into organic molecules. The enzyme RuBisCO is responsible for transformation of inorganic carbon into organic compounds in case of so called C3-plants, which represent 90 % of all 300 000 until now known plant species, the residual 10 % of plants has in addition to RuBisCO also other alternative enzyme pathways serving for fixation of carbon dioxide (CO₂), but these pathways serve only to the purpose of increasing the concentration of CO₂ in the neighbourhood of the enzyme RuBisCO. RuBisCO forms within the Calvin cycle 3-phosphoglycerate. From 3-phosphoglycerate is formed glyceraldehyde-3-phosphate, serving as starting compound for further formation of glucose, starch and other compounds (Malkin R, Niyogi K. Photosynthesis. In book: Biochemistry and Molecular Biology of Plants. Buchanan B.B., Gruissem W., Jones R.L. (Eds.), American Society of Plant Physiologists, Rockville, MD, USA, chapter 12, 568-628 (2000); Raghavendra A. S. Photosynthesis and partitioning in C3 plants. In book: Encyclopedia of Applied Plant Science. Thomas B., Murphy D.J., Murray B.G., (Eds.), Elsevier, Oxford, 673-680 (2003)).

For the reasons mentioned above, changes in activity of the enzyme as well as in its content are of importance. Plant conversion of inorganic carbon into organic matter is very important for global cycling of carbon. The capacity and the yield of photosynthesis are influenced by illumination, temperature, and the concentration of available CO₂. Except for the ability to bind CO₂, RuBisCO acts as oxygenase, too, which rather decreases the photosynthesis yield but it enables the plant to synthesize glycine and serine. The ratio of those activities is controlled by the ration of partial pressures of CO₂ and O₂.

RuBisCO is a complex protein made up of 8 large subunits (about 50 000 Da each) and 8 small subunits (about 14 000 Da each). The large subunit is encoded for in the plastid genome, whereas the small subunit in the nucleus. Within photosynthesis, the solar energy is converted into chemical energy represented by molecules of NADPH and ATP. During CO₂ fixation by RuBisCO, both these compounds are consumed.

Photosynthesis in C3-plants is regulated in many ways. For instance, plant hormones, such as brassinosteroids, are known to positively influence the photosynthesis yield (Hradecká, Kohout & Holá, Kohout - unpublished results). During photosynthesis the energy of light is converted into chemical energy in the form of NADPH and ATP. During CO₂ fixation by the enzyme RuBisCO within the Calvin cycle these compounds are consumed. For the enzyme to work properly, Mg²⁺ ions need to be available. The activity of RuBisCO and its turn-over influence CO₂ fixation efficiency; for instance, RuBisCO level in plants decreases with the lack of nitrogen and thus the photosynthetic fixation of CO₂ is limited. RuBisCO is inactive in the dark; it requires illumination to become active. The activation is a result of carbamylation, which consists of binding of CO₂ and Mg²⁺ onto lysine residue in the active centre closeness. The carbamylation changes the conformation of the large subunit and activates the enzyme while Mg2+ stabilizes the active conformation. RuBisCO is thus only active when lysine 201 reacts with CO₂ in the active centre nearness, allowing Mg²⁺ to bind. Noncarbamylated enzyme binds ribulose 1,5-bisphosphate (RuBP) too tightly for the catalytic reaction to occur. However, RuBisCO can be inhibited by some compounds such as 2-carboxyarabinitol-1-phosphate (CA1P).

Another protein, RuBisCO activase, releases inhibitors (e.g. CA1P) that bind to the active site. The inhibitors hinder the activation (carbamylation) of the enzyme in the dark. RuBisCO activase is activated by light by means of ATP. The activation of RuBisCO and its activase by light represent the next mechanism of important light regulation of photosynthetic CO₂ assimilation.

CA1P, a compound naturally present in the leaves of many plants, is a strong inhibitor of RuBisCO. The affinity of the enzyme to CA1P is much higher than to the substrate. CA1P accumulates in the dark and blocks the active sites, and is released after illumination or after RuBisCO activase action. However, it was not found in all plants. RuBisCO and other cooperating enzymes are activated by the increase of pH and Mg²⁺ concentration; they are almost inactive at pH 7.2.

The significance of influencing RuBisCO activity consists in the ability to increase the yield of photosynthesis. Most agriculture crops (cereals, legumes, oil plants) belong to plants with C3 metabolism. Three main factors limit the photosynthetic yield; losses by photorespiration (which is an unavoidable result of oxygenase activity of RuBisCO), high water requirements, and temperate zone preference. A known factor that increases the efficiency of photosynthetic CO₂ fixation is an increased level of CO₂. RuBisCO is a rather slow-working enzyme; one molecule is able to fix only three molecules of CO₂ per second, thus each increase of reaction rate is significant in global effectivity of the process.

Thus influencing the enzyme in the way that would lead to a higher activity is of particular significance for the increase in the photosynthetic yield, which is important primarily in the agriculture, as well as for the increase in the fixation of atmospheric CO₂, hence in fighting high CO₂ concentrations in the atmosphere.

### Summary of the invention

Inventors of this invention have found out that some low-molecular organic compounds influence the activity of RuBisCO. Under standard conditions, it has been discovered that these compounds increase the CO₂ fixation by RuBisCO *in vitro* by more than 10% on average. It also represents increase in organic matter formation alike as well as significant fixation of CO₂ from the atmosphere.

As an example, ecdysteroids can be stated. Ecdysteroids belong to plant oxysterols whose hormonal effect on insects had been proved. Their function in plants has been unknown yet. We discovered that these compounds influence the carboxylase activity of the enzyme. The active enzyme consists of 8 large subunits and 8 small subunits considered to have regulatory role. We identified the small subunits as proteins able to bind 20-hydroxyecdysone (20HE) in bioaffinity chromatography. Further testing revealed the affinity of related oxysterols to both subunits of RuBisCO.

In a similar way, also other oxysterols act. These include brassinosteroids, plant hormones that influence plant growth and particularly are involved in suppressing stress response in plants.

When assaying carboxylase activity of RuBisCO with the use of ¹⁴C-labeled CO₂, molar ratio of the steroid to enzyme 1:1 did not show significant increase in activity. However, when the molar ratio was 10:1, the increase in activity is higher than 10%. When twenty fold excess of steroid is applied, no further increase appears.

The subject matter of the invention is therefore a method of increasing of photosynthetic assimilation of carbon dioxide by the influence the carboxylase activity of the enzyme RuBisCO leading to an increase of the biomass of agricultural products and to a lowering of the carbon dioxide content in the atmosphere, wherein at least one low molecular weight steroid of both natural and synthetic origin is applied in the molar ratio from 10:1 to 20:1 to the enzyme RuBisCO.

After analytical assay was optimized and the hypothesis has been verified that 20-hydroxyecdysone is able to influence the carboxylase activity of RuBisCO, screening of available steroids and other compounds was carried out in order to find out whether they are able to influence the carboxylase activity of RuBisCo as well.

### Examples

### Example 1

### Effect of 20-hydroxyecdysone (20E) on the yield of RuBisCO carboxylase reaction.

Relative amount of product detected represents the ratio of the amount of [1-14C]-3-phosphoglycerate produced by the reaction in the presence of 20E to the amount of [1-14C]-3-phosphoglycerate produced by the reaction in no presence of 20E.

The influence of ecdysteroids on RuBisCO assay was based on the protocol described by Pierce et al. (Pierce JW, McCurry SD, Mulligan RM, Tolbert NE. Activation and assay of ribulose-1,5-bisphosphate carboxylase/oxygenase. Methods Enzymol. 1982; 89: 47-55.), where the RuBisCO carboxylase activity is assayed by the rate of formation of acid-stable ¹⁴C product, [1-⁴C]-3-phosphoglycerate, formed from NaH¹⁴CO₃. First, the enzyme from spinach was activated at 2 mg.mL⁻¹ in the activating buffer - 100 mM Bicine pH 8.2, non-labelled 10 mM NaHCO₃, 20 mM MgCl₂, 0.2 mM EDTA and 1 mM dithiodireitol - for ten minutes at 30 °C. The assay itself was carried out in 20 ml scintillation vials in a solution prepared by mixing 250 µL of 200 mM bicine buffer pH 8.2, 0.4 mM EDTA and 1 mM dithiothreitol, 5 µL of 2 M MgCl₂, 150 µL of 20E solution or water (in the case of the control), 20 µL of 250 mM NaH¹⁴CO₃ solution and 20 µL of 12.5 mM ribulose-1,5-bisphosphate. 250 mM NaH¹⁴CO₃ was prepared by mixing NaH¹⁴CO₃ solution (specific activity 2 mCi/ml) with 0.3 M NaHCO₃ in the ratio of 1:4. The reaction was initiated by the addition of 100 µL of the activated enzyme and the vial was swirled so that the solution components could mix. The reaction was stopped by the addition of 200 µL of 2 M HCl. To remove excess ¹⁴CO₂ and acid, the vials were slowly heated at 95 °C in a hood. After the liquid evaporated and the vials were cooled at laboratory temperature, 5 mL of scintillation solution Rotiszint^{®} eco plus was added. The concentration of 20E was chosen so that its final molar concentration to the molar concentration of the enzyme in the vial was 1:1; the stock solution containing 1 mg of 20E per ml was diluted to the desired concentration just before the experiment. Results in Table 1.

**Table 1**

| Relative amount of product detected | |
|---|---|
| Without steroid | 1 |
| 20-hydroxyecdysone (1:1)* | 1.05 |

| | |
|---|---|
| *Numbers in brackets represent the molar ratio of steroid to enzyme - 1:1 means that I mg of the enzyme was in the mixture with 8,75.10⁻⁴ mg of 20-hydroxyecdysone. | |

### Example 2

Relative amount of product detected in the presence of different amounts of 20E to the amount in no presence of 20E.

The same experiment as in Example 1 was carried out but different amounts of 20-hydroxyecdysone were used. Results in Table 2.

**Table 2**

| Relative amount of product detected | |
|---|---|
| without steroid | 1.00 |
| 20-hydroxyecdysone (1:1)* (see example 1) | 1.05 |
| 20-hydroxyecdysone (10:1)* | 1.13 |
| 20-hydroxyecdysone (20:1)* | 1.12 |

| | |
|---|---|
| * Numbers in brackets represent the molar ratio of steroid to enzyme - 10:1 means that 10fold excess of steroid over RuBisCO was used. | |

Results in Table 2 show that 10fold increase in ecdysteroid amount resulted in the increase of carboxylase activity. However, further increase did not show further increase in the activity of the enzyme.

### Example 3

Relative amount of product detected in the presence of different brassinosteroids to the amount in no presence of them.

The same experiment as in Example 1 was carried out but different brassinosteroids were used. Results in Table 3.

**Table 3**

| Relative amount of product detected | |
|---|---|
| Without steroid | 1.00 |
| 24-epibrassinolide (10:1) | 1.10 |
| 24-epicastasterone (10:1) | 1.20 |

### Example 4

Relative amount of product detected in the presence of different ecdysteroids to the amount in no presence of them.

The same experiment as in Example 1 was carried out but different ecdysteroids were used. Results in Table 4.

**Table 4**

| Relative amount of product detected | |
|---|---|
| Without steroid | 1.00 |
| Ajugasterone C (10:1) | 1.13 |
| Polypodine B (10:1) | 1.11 |

### Example 5

Relative amount of product detected in the presence of mixed ecdysteroids to the amount in no presence of them.

The same experiment as in Example 1 was carried out but mixed ecdysteroid fractions were used. Results in Table 5.

**Table 5**

| Relative amount of product detected | |
|---|---|
| Without steroid | 1.00 |
| 80% 20-hydroxyecdysone (10:1) | 1.11 |
| Ecdysteroidal fraction from root extracts of *Leuzea carthamoides* | 1.13 |

### Example 6

### Steroids influence on overall activity of photosynthesis in vivo

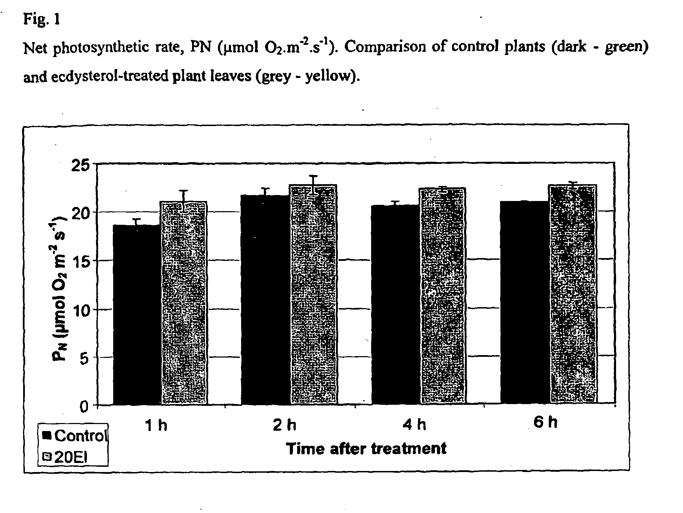

Activity of photosynthesis was measured as the production of oxygen by circular target (average 35 mm) cut from the third leaf (counted from the top of the plant) that was placed in the sampling chamber. Assay: oxygen electrode (Clark electrode), sampling chamber LD2 (Hansatech, King's Lynn, Great Britain). Target was incubated in the dark for 5 minutes followed by 9 minutes of illumination; production of oxygen evaluated in the fifth-eighth minute after illumination (in the linear part of the light curve). Illumination was 240 micromol.m⁻².s⁻¹. Plants were 96 days old when analyzed (96 days after sowing). The ratio of 20E to enzyme was 1:10, thus the most effective from Example 2. Results are presented in Table 6 and Figure 1.

**Table 6**

| Net photosynthetic rate. PN (µmol O₂.m⁻².s⁻¹) | | | | | | | | |
|---|---|---|---|---|---|---|---|---|
| Plant nr. | 1 hour | | 2 hours | | 4 hours | | 6 hours | |
| | Control | 20E | Control | 20E | Control | 20E | Control | 20E |
| 1 | 15.89 | 17.02 | 19.29 | 18.73 | 20.43 | 21.56 | 21.00 | 23.83 |
| 2 | 18.16 | 21.56 | 21.00 | 21.56 | 22.13 | 22.70 | 21.19 | 22.70 |
| 3 | 19.29 | 22.70 | 22.70 | 23.83 | 21.19 | 22.31 | 20.64 | 22.31 |
| 4 | 18.96 | 18.40 | 22.31 | 23.42 | 18.96 | 21.75 | 21.19 | 21.75 |
| 5 | 18.96 | 23.42 | 23.42 | 25.10 | 20.64 | 22.31 | 20.64 | 23.42 |
| 6 | 20.64 | 23.42 | | 23.98 | 20.64 | 23.42 | | 22.31 |
| **Average** | **18.65** | **21.09** | **21.74** | **22.77** | **20.67** | **22.34** | **20.93** | **22.72** |
| **SD** | **1.58** | **2.74** | **1.63** | **2.29** | **1.04** | **0.67** | **0.28** | **0.77** |
| **SEM** | **0.64** | **1.12** | **0.73** | **0.93** | **0.42** | **0.27** | **0.12** | **0.32** |

Table 6 as well as Figure 1 show that statistically evidential differences were found 4 and 6 hours after treatment. Less evidential difference was achieved after 1 hour.

When monitoring steroids effect, statistically evidential differences decrease, however, increase in activity dies down after 50 hours.

### Industrial applicability

Applicability of the invention lies in the increase of green matter (due to increase in carboxylase activity of the enzyme) as well as in the increase of CO₂ assimilation, which actually could be even more important. Thus the invention can be considered as an effective means of greenhouse gases fighting as 10% increase in the activity of the enzyme entails great contribution in CO₂ decrease from the atmosphere.

## Claims

1. A method of increasing of photosynthetic assimilation of carbon dioxide by influencing the carboxylase activity of the enzyme RuBisCO leading to an increase of the biomass of agricultural products and to lowering of the carbon dioxide content in the atmosphere, wherein at least one low molecular weight steroid of both natural and synthetic origin is applied in the molar ratio from 10:1 to 20:1 to the enzyme RuBisCO.

2. The method as claimed in claim 1, wherein steroids are ecdysteroids.

3. The method as claimed in claim 1, wherein steroids are brassinosteroids.

4. The method as claimed in claim 2, wherein ecdysteroid is 20-hydroxyecdysone.

5. The method as claimed in claim 3, wherein brassinosteroids are natural brassinosteroids.

6. The method as claimed in claim 5, wherein natural brassinosteroid is 24-epicastasterone.

7. The method as claimed in claim 1, wherein steroids are ajugasterone C or polypodine B.

8. The method as claimed in claim 4, wherein ecdysteroid is raw extract or a mixture of steroids.

## Patentansprüche

1. Verfahren zur Erhöhung der photosynthetischen Assimilation von Kohlendioxid durch Beeinflussung der Carboxylase Aktivität des Enzyms RuBisCO zu einer Vermehrung der Biomasse der landwirtschaftlichen Produkte und zu einer Verringerung des Kohlendioxidgehaltes in der Atmosphäre, wobei mindestens ein Niedermolekulargewicht-Steroid sowohl natürlichen als auch synthetischen Ursprungs in einem molaren Verhältnis von 10:1 1 bis 20:1 an das Enzym RuBisCO angebracht wird.

2. Verfahren nach Anspruch 1, in dem Steroide Ecdysteroide sind.

3. Verfahren nach Anspruch 1, in dem Steroide Brassinosteroide sind.

4. Verfahren nach Anspruch 2, in dem das Ecdysteroid 20-Hydroxyecdyson ist.

5. Verfahren nach Anspruch 3, in dem Brassinosteroide natürliche Brassinosteroide sind.

6. Verfahren nach Anspruch 5, in dem natürliches Brassinosteroid 24-epicastasteron ist.

7. Verfahren nach Anspruch 1, in dem Steroide Ajugasterone C oder Polypodine sind.

8. Verfahren nach Anspruch 4, in dem Ecdysteroid ein Rohextrakt oder ein Gemisch von Steroiden ist.

## Revendications

1. Procédé d'augmentation de l'assimilation photosynthétique du dioxyde de carbone en agissant sur l'activité de la carboxylase de l'enzyme RuBisCO conduisant à une augmentation de la biomasse des produits agricoles et d'abaissement de la teneur en dioxyde de carbone dans l'atmosphère, dans lequel au moins une faible stéroïde de poids moléculaire d'origine à la fois naturelle et synthétique est appliquée dans le rapport molaire 10:01 - 20:01 à l'enzyme RuBisCO.

2. Procédé selon la revendication 1, dans lequel les stéroïdes sont ecdystéroïdes.

3. Procédé selon la revendication 1, dans lequel les stéroïdes sont des brassinostéroïdes.

4. Procédé selon la revendication 2, dans lequel ecdystéroïde est 20-hydroxyecdysone.

5. Procédé selon la revendication 3, dans lequel les brassinostéroïdes sont brassinostéroïdes naturelles.

6. Procédé selon la revendication 5, dans lequel brassinostéroïde naturel est de 24-epicastasteron.

7. Procédé selon la revendication 1, dans lequel les stéroïdes sont ajugastérone C ou polypodine B.

8. Procédé selon la revendication 4, dans lequel ecdystéroïde est extrait brut ou un mélange de stéroïdes.
